(19) Europäisches Patentamt
European Patent Office
Office européen des brevets

(11) **EP 4 356 933 A1**

(12) **EUROPEAN PATENT APPLICATION**
published in accordance with Art. 153(4) EPC


(43) Date of publication:
**24.04.2024 Bulletin 2024/17**

(21) Application number: **23814344.0**

(22) Date of filing: **06.09.2023**

(51) International Patent Classification (IPC):
***A61K 48/00*** *(2006.01)* ***A61K 9/127*** *(2006.01)*
***A61K 39/00*** *(2006.01)* ***A61P 35/00*** *(2006.01)*
***A61P 37/00*** *(2006.01)* ***A61P 29/00*** *(2006.01)*
***A61P 31/12*** *(2006.01)* ***A61P 31/04*** *(2006.01)*
***A61K 8/60*** *(2006.01)* ***A61K 8/14*** *(2006.01)*

(86) International application number:
**PCT/KR2023/013302**

(87) International publication number:
**WO 2024/054020 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **07.09.2022 KR 20220113631**
**07.08.2023 KR 20230102612**



(71) Applicant: **Eyegene, Inc.**
**Seoul 07528 (KR)**

(72) Inventors:
- **CHO, Yang Je**
**Yongsan-gu, Seoul 04423 (KR)**
- **KIM, Seok Hyun**
**Goyang-si Syeonggi-do 10416 (KR)**
- **KIM, Kwangsung**
**Goyang-si Gyeonggi-do 10371 (KR)**

(74) Representative: **Mewburn Ellis LLP**
**Aurora Building**
**Counterslip**
**Bristol BS1 6BX (GB)**


Remarks:
The complete document including Reference Table(s) and the Sequence Listing(s) can be downloaded from the EPO website

(54) **COMPOSITION FOR DELIVERING MODIFIED NUCLEIC ACID-CONTAINING MRNA**


(57) Disclosed is a composition for delivering mRNA containing a modified nucleotide including liposomes based on a cationic lipid. The composition for delivering mRNA containing a modified nucleotide has superior storage stability and high expression efficiency *in vivo,* thus increasing the stability and efficiency of mRNA vaccines for cancer treatment or mRNA vaccines for preventing viral infections and other mRNA vaccines or therapeutics.


**Figure 1**

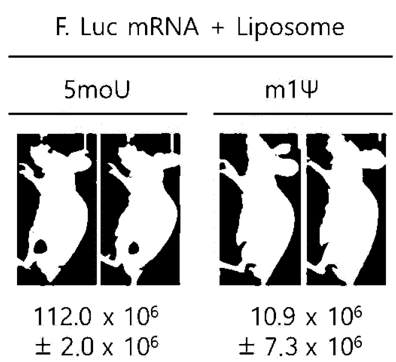


EP 4 356 933 A1

## Description

BACKGROUND OF THE INVENTION

Field of the Invention

The present invention relates to a composition for *in-vivo* delivering mRNA containing a modified nucleotide, and more particularly to a composition for *in-vivo* delivering mRNA containing a modified nucleotide, which includes liposomes based on a cationic lipid.

Description of the Related Art

Gene therapy and genetic vaccination are technologies that have already been proven in the field of medicine and are generally applied, and may be used to treat not only genetic diseases, but also autoimmune diseases, infectious diseases, cancer or tumorrelated diseases, inflammatory diseases, and the like.

Genetic vaccines began to be developed since it was reported that, when DNA and RNA encoding a target gene are directly injected into an animal, the target gene is expressed in the living animal, and immunity is possible by this expression (Wolff JA et al. Science, 247:1465-8, 1990).

Genetic vaccination evokes the desired immune response against selected antigens, such as characteristic components of bacterial surfaces, viral particles, tumor antigens, and the like. All in all, vaccination is one of the pivotal achievements of modern medicine. However, effective vaccines are currently only available for a limited number of diseases. Thus, infections that cannot be prevented by vaccination still affect millions of people each year.

In gene therapy or genetic vaccination, DNA and RNA may be used as nucleic acid molecules for gene administration, and it is known that DNA is relatively stable and easy to handle compared to RNA. However, in case of DNA, a potential risk may arise if the DNA segment administered into the patient's genome is inserted at an undesirable location and the gene is damaged. Additionally, unwanted anti-DNA antibodies may appear, and another problem is that the level of the peptide or protein expressed by transcription/translation after DNA administration is limited. The presence or absence of a specific transcription factor that regulates DNA transcription has a major impact on the expression level of the administered DNA, and in the absence of a specific transcription factor, a sufficient amount of RNA is not produced by DNA transcription, and consequently, the level of peptide or protein produced by translation is also limited.

On the other hand, when RNA is used as a tool for gene administration, RNA does not require transcription and is thus able to directly synthesize proteins in the cytoplasm without the need to enter the nucleus like DNA, so there is no fear of intruding into the cell chromosome and causing unwanted genetic damage. Moreover, RNA has a shorter half-life than DNA and thus does not induce longterm genetic modification (Sayour EJ, et al., J. Immunother. Cancer 2015; 3:13, 2015). When delivered into cells, a general RNA vaccine is activated for a short period of time to express a target protein and is destroyed by an enzymatic reaction within a few days, and a specific immune response to the expressed target antigen (protein) remains.

In addition, when using RNA as a tool for gene administration, RNA works only when it passes through the cell membrane without the need to pass through the nuclear membrane, making it possible to express the target protein in a similar level as when using DNA even in a smaller amount than DNA. Moreover, RNA itself has immunereinforcing activity and is thus capable of exhibiting the same immune effect even when administered in a small amount compared to DNA.

By using RNA instead of DNA for genetic vaccination, the risk of unwanted integration into the genome and the production of anti-DNA antibodies may be minimized or avoided. However, RNA is a highly unstable molecular species that may be readily degraded by ubiquitous RNases.

Although many advances have been made in the past years, there remains a need in the art to provide a method in which the effectiveness of administration is not compromised by inefficient translation of mRNA due to premature degradation of antigen or inefficient release of mRNA from cells, as an efficient method for mRNA vaccination that may induce an immune response. Furthermore, there is also a need to reduce the dose of mRNA vaccines in order to reduce potential safety concerns and to make vaccines affordable throughout the third world.

There are still many issues to be solved with regard to nucleic acid delivery to obtain a desired response in a biological system. Nucleic acid-based therapeutics hold tremendous promise, but realizing this promise requires effective delivery of nucleic acids to appropriate regions within cells or organisms.

However, the use of nucleic acids for therapeutic and prophylactic purposes currently faces two problems. First, free RNA is susceptible to degradation by nucleases in blood. Second, free RNA has limited ability to access intracellular compartments where the translational machinery that translates the same resides. Incorporating lipid nanoparticles formed from cationic lipids and other lipid components such as neutral lipids, cholesterol, PEG, pegylated

lipids, and oligonucleotides has been attempted to block degradation of RNA in blood and promote cellular uptake of nucleic acids.

When a delivery carrier based on lipids such as liposomes or lipid nanoparticles is used, mRNA is usually adsorbed outside or encapsulated inside. In particular, when mRNA is adsorbed to the outside of the delivery carrier, it is known to use an aggregate of liposomes and a nucleic acid, rather than liposomes alone. Since the combination of lipids, the state of the nucleic acid, and the ratio of nucleic acid to liposomes may affect adsorption capacity and stability, optimization therefor is required.

In general, also, even when *in-vitro* mRNA expression of the delivery carrier is verified, there is a problem in that the results of *in-vivo* expression thereof may be different. There are factors that reduce intracellular delivery efficiency, such as half-life and bio-distribution reduction due to protein aggregation in blood, different cellular uptake methods based on the lipid composition, barrier by ECM (extracellular matrix), etc., depending on the composition of lipids and the physicochemical properties of the delivery carrier *in vivo.*

Meanwhile, in order to increase the efficiency of expression of mRNA delivered *in vivo,* methods of using mRNA constructed by modifying the base of mRNA have been attempted, and it is known that a modified nucleotide has high expression efficiency *in vivo* compared to mRNA using an unmodified nucleotide (US8278036B2, KR10-2171849B1, KR10-2014601B1). Examples of the modified nucleotide may include pseudouridine, N1-methyl-pseudouridine, etc., but it is necessary to optimize the combination of the delivery carrier and the modified nucleotide.

Accordingly, the present inventors have made great efforts to find techniques for improving a delivery carrier capable of inducing stable protein expression by stably delivering mRNA containing a modified nucleotide *in vivo* to increase intracellular expression efficiency, and thus ascertained that [liposome + mRNA complex] based on cationic liposomes and mRNA containing a specific modified nucleotide may exhibit low inflammatory response and high expression efficiency *in vivo,* thereby culminating in the present invention.

## SUMMARY OF THE INVENTION

It is an object of the present invention to provide a composition for delivering mRNA containing a modified nucleotide, which includes liposomes based on a cationic lipid, with low inflammatory response and high expression efficiency *in vivo.*

It is another object of the present invention to provide a pharmaceutical composition for preventing or treating a disease selected from the group consisting of cancer, a tumor, an autoimmune disease, a genetic disease, an inflammatory disease, a viral infection, and a bacterial infection, including the composition for delivering mRNA containing a modified nucleotide described above as an active ingredient.

It is still another object of the present invention to provide a vaccine including the composition for delivering mRNA containing a modified nucleotide described above as an active ingredient.

It is yet another object of the present invention to provide a functional cosmetic composition including the composition for delivering mRNA containing a modified nucleotide described above as an active ingredient.

In order to accomplish the above objects, the present invention provides a composition for delivering mRNA containing a modified nucleotide, including liposomes based on a cationic lipid.

In addition, the present invention provides a pharmaceutical composition for preventing or treating a disease selected from the group consisting of cancer, a tumor, an autoimmune disease, a genetic disease, an inflammatory disease, a viral infection, and a bacterial infection, including the composition for delivering mRNA containing a modified nucleotide described above as an active ingredient.

In addition, the present invention provides a vaccine including the composition for delivering mRNA containing a modified nucleotide described above as an active ingredient.

In addition, the present invention provides a functional cosmetic composition including the composition for delivering mRNA containing a modified nucleotide described above as an active ingredient.

In addition, the present invention provides a method of preventing or treating a disease selected from the group consisting of cancer, a tumor, an autoimmune disease, a genetic disease, an inflammatory disease, a viral infection, and a bacterial infection, including administering a composition for delivering mRNA containing a modified nucleotide including liposomes based on a cationic lipid.

In addition, the present invention provides the use of a composition for delivering mRNA containing a modified nucleotide including liposomes based on a cationic lipid for preventing or treating a disease selected from the group consisting of cancer, a tumor, an autoimmune disease, a genetic disease, an inflammatory disease, a viral infection, and a bacterial infection.

In addition, the present invention provides the use of a composition for delivering mRNA containing a modified nucleotide including liposomes based on a cationic lipid for the manufacture of a medicament for preventing or treating a disease selected from the group consisting of cancer, a tumor, an autoimmune disease, a genetic disease, an inflammatory disease, a viral infection, and a bacterial infection.

## BRIEF DESCRIPTION OF THE DRAWINGS

The above and other objects, features, and other advantages of the present invention will be more clearly understood from the following detailed description taken in conjunction with the accompanying drawings, in which:

FIG. 1 shows mRNA expression efficiency in mice depending on the type of modified nucleotide in mRNA-liposome complexes; and

FIG. 2 shows results of RBD-specific IgG antibody titer analyzed by ELISA in serum of mice administered mRNA-liposome complexes prepared depending on the type of modified nucleotide.

## DETAILED DESCRIPTION OF THE INVENTION

Unless otherwise defined, all technical and scientific terms used herein have the same meanings as typically understood by those skilled in the art to which the present invention belongs. In general, the nomenclature used herein is well known in the art and is typical.

In *in-vivo* gene transfer using a nucleic acid, with the development of a method of preventing or treating a disease by inducing desired immune responses against selected antigens such as characteristic components of viral or bacterial surfaces, virus particles, and tumor antigens and inducing expression of therapeutic proteins, the present invention is intended to provide a method that exhibits a stable effect by increasing intracellular expression efficiency of mRNA. Therefore, a composition for delivering mRNA using liposomes based on a cationic lipid is prepared, confirming low inflammatory response and high expression efficiency *in vivo.*

In particular, performance of the composition for delivering mRNA containing a specific type of modified nucleotide is confirmed to be vastly superior.

In the composition for delivering mRNA using liposomes based on a cationic lipid according to the present invention, mRNA may be provided in the form of a complex with liposomes based on a cationic lipid, and accordingly, the composition for delivering mRNA using liposomes based on a cationic lipid is interchangeably used with [liposome + mRNA complex] .

Hereinafter, a detailed description will be given of the present invention.

An aspect of the present invention pertains to a composition for delivering mRNA containing a modified nucleotide, which includes liposomes based on a cationic lipid.

In the present invention, the modified nucleotide may include at least one backbone-modified, sugar-modified, or base-modified nucleotide, but is not limited thereto.

### Sugar modification:

A modified nucleoside or nucleotide that may be incorporated into a modified mRNA compound including mRNA sequences as described herein may be modified at the sugar moiety. For example, a 2' hydroxyl group (OH) may be modified or replaced with a number of different "oxy" or "deoxy" substituents. Examples of "oxy"-2' hydroxyl group modifications include, but are not limited to, alkoxy or allyloxy (-OR, for example, R = H, alkyl, cycloalkyl, aryl, aralkyl, heteroaryl, or sugar); polyethylene glycol (PEG), $-O(CH_2CH_2O)_nCH_2CH_2OR$; "locked" nucleic acid (LNA) in which 2' hydroxyl is linked to the 4' carbon of the same ribose sugar by, for example, a methylene crosslinkage; and an amino group (-O-amino, in which the amino group, for example, NRR is an alkylamino, dialkylamino, heterocyclyl, arylamino, diarylamino, heteroarylamino, diheteroarylamino, ethylenediamine, or polyamino) or aminoalkoxy.

The "deoxy" modification may include hydrogen or amino (e.g. $NH_2$; alkylamino, dialkylamino, heterocyclyl, arylamino, diarylamino, heteroarylamino, diheteroarylamino, or amino acid); or an amino group may be attached to the sugar through a linker, in which the linker includes at least one selected from among atoms C, N, and O.

The sugar group may also contain at least one carbon in the opposite stereochemical configuration compared to the corresponding carbon in ribose. Thus, modified mRNA may include a nucleotide containing arabinose, such as sugar.

### Backbone modification:

In a modified nucleoside or nucleotide that may be incorporated into a modified mRNA compound including mRNA sequences as described herein, the phosphate backbone may be further modified. The phosphate group of the backbone may be modified by replacing one or more oxygen atoms with other substituents. Also, a modified nucleoside or nucleotide may include the complete replacement of an unmodified phosphate moiety with a modified phosphate described herein. Examples of the modified phosphate group may include, but are not limited to, phosphorothioate, phosphoroselenate, boranophosphate, boranophosphate ester, hydrogen phosphonate, phosphoroamidate, alkyl or aryl

phosphonate, and phosphotriester. Phosphorodithioate has both unlinked oxygens replaced by sulfur.
A phosphate linker may also be modified by substitution of linked oxygen with nitrogen (crosslinked phosphoroamidate), sulfur (crosslinked phosphorothioate), or carbon (crosslinked methylenephosphonate).

Base modification:

A modified nucleoside or nucleotide that may be incorporated into a modified mRNA compound including mRNA sequences as described herein may be further modified at the nucleobase moiety. Examples of the nucleobase found in mRNA include, but are not limited to, adenine, guanine, cytosine, and uracil. For example, the nucleoside or nucleotide described herein may be chemically modified at the major groove face. In some embodiments, major groove chemical modifications may include amino groups, thiol groups, alkyl groups, or halo groups.
In a preferred embodiment of the present invention, the nucleotide analogue/modification is selected from among base modifications such as 2-amino-6-chloropurine riboside-5'-triphosphate, 2-aminopurine-riboside-5'-triphosphate, 2-aminoadenosine-5'-triphosphate, 2'-amino-2'-deoxycytidine-triphosphate, 2-thiocytidine-5'-triphosphate, 2-thiouridine-5'-triphosphate, 2'-fluorothymidine-5'-triphosphate, 2'-O-methyl-inosine-5'-triphosphate, 4-thiouridine-5'-triphosphate, 5-aminoallylcytidine-5'-triphosphate, 5-aminoallyluridine-5'-triphosphate, 5-bromocytidine-5'-triphosphate, 5-bromouridine-5'-triphosphate, 5-bromo-2'-deoxycytidine-5'-triphosphate, 5-bromo-2'-deoxyuridine-5'-triphosphate, 5-iodocytidine-5'-triphosphate, 5-iodo-2'-deoxycytidine-5'-triphosphate, 5-iodouridine-5'-triphosphate, 5-iodo-2'-deoxyuridine-5'-triphosphate, 5-methylcitidine-5'-triphosphate, 5-methyluridine-5'-triphosphate, 5-propynyl-2'-deoxycytidine-5'-triphosphate, 5-propynyl-2'-deoxyuridine-5'-triphosphate, 6-azacytidine-5'-triphosphate, 6-azauridine-5'-triphosphate, 6-chloropurine riboside-5'-triphosphate, 7-deazaadenosine-5'-triphosphate, 7-deazaguanosine-5'-triphosphate, 8-azaadenosine-5'-triphosphate, 8-azidoadenosine-5'-triphosphate, benzimidazole-riboside-5'-triphosphate, N1-methyladenosine-5'-triphosphate, N1-methylguanosine-5'-triphosphate, N6-methyladenosine-5'-triphosphate, O6-methylguanosine-5'-triphosphate, pseudouridine-5'-triphosphate, puromycin-5'-triphosphate, and xanthosine-5'-triphosphate.
Preferred is any base-modified nucleotide selected from the group consisting of 5-methylcytidine-5'-triphosphate, 7-deazaguanosine-5'-triphosphate, 5-bromocytidine-5'-triphosphate, and pseudouridine-5'-triphosphate.
In some embodiments, the modified nucleoside may include pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyl-uridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, or 4-methoxy-2-thio-pseudouridine.
In some embodiments, the modified nucleoside may include 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, or 4-methoxy-1-methyl-pseudoisocytidine.
In other embodiments, the modified nucleoside may include 2-aminopurine, 2,6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentyladenosine, N6-(cis-hydroxyisopentyl)adenosine, 2-methylthio-N6-(cis-hydroxyisopentenyl)adenosine, N6-glycinylcarbamoyladenosine, N6-threonylcarbamoyladenosine, 2-methylthio-N6-threonylcarbamoyladenosine, N6,N6-dimethyladenosine, 7-methyladenine, 2-methylthio-adenine, or 2-methoxy-adenine.
In other embodiments, the modified nucleoside may include inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, or N2,N2-dimethyl-6-thio-guanosine.
In some embodiments, the nucleotide may be modified at the major groove face and may include replacement of hydrogen with a methyl group or halo group at C-5 of uracil. In certain embodiments, the modified nucleoside is 5'-O-(1-thiophosphate)-adenosine, 5'-O-(1-thiophosphate)-cytidine, 5'-O-(1-thiophosphate)-guanosine, 5'-O-(1-thiophosphate)-uridine, or 5'-O-(1-thiophosphate)-pseudouridine.
In a further embodiment, the modified mRNA may include any nucleoside modification selected from among 6-aza-cytidine, 2-thio-cytidine, α-thio-cytidine, pseudoisocytidine, 5-aminoallyl-uridine, 5-iodo-uridine, N1-methyl-pseudouridine, 5,6-dihydrouridine, α-thio-uridine, 4-thio-uridine, 6-aza-uridine, 5-hydroxy-uridine, deoxy-thymidine, 5-methyl-uridine, pyrrolo-cytidine, inosine, α-thio-guanosine, 6-methyl-guanosine, 5-methyl-cytidine, 8-oxo-guanosine, 7-deaza-guanosine, N1-methyl-adenosine, 2-amino-6-chloro-purine, N6-methyl-2-amino-purine, 6-chloro-purine, N6-methyl-adenosine, α-thio-adenosine, 8-azido-adenosine, and 7-deaza-adenosine.

In the present invention, the modified nucleotide is selected from the group consisting of pseudouridine (Ψ), N1-methylpseudouridine (m1Ψ), 5-methyluridine (m5U), 2-thiouridine (s2U), 2'-O-methyl-uridine (Um), 5-methylcytidine (m5C), and 5-methoxyuridine (5moU), and is most preferably 5-methoxyuridine (5moU), but is not limited thereto.

In the present invention, the cationic lipid includes a lipid that is continuously cationic without the influence of pH change or an ionic lipid that is converted to be cationic by pH change.

In the present invention, the cationic lipid is preferably at least one selected from the group consisting of dimethyldioctadecylammonium bromide (DDA), C12-200, 1,2-dioleoyl-3-trimethylammonium propane (DOTAP), 3β-[N-(N',N'-dimethylaminoethane)carbamoyl cholesterol (DC-Chol), 1,2-dioleoyloxy-3-dimethylammonium propane (DODAP), 1,2-di-O-octadecenyl-3-trimethylammonium propane (DOTMA), 1,2-dimyristoleoyl-sn-glycero-3-ethylphosphocholine (14:1 Ethyl PC), 1-palmitoyl-2-oleoyl-sn-glycero-3-ethylphosphocholine (16:0/18:1 Ethyl PC), 1,2-dioleoyl-sn-glycero-3-ethylphosphocholine (18:1 Ethyl PC), 1,2-distearoyl-sn-glycero-3-ethylphosphocholine (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholine (12:0 Ethyl PC), N1-[2-((1S)-1-[(3-aminopropyl)amino]-4-[di(3-aminopropyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,N-dimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TAP), 1,2-dimyristoyl-3-trimethylammonium-propane (14:0 TAP), and N4-cholesteryl-spermine (GL67), but is not limited thereto, and is most preferably C12-200 or 1,2-dioleoyl-3-trimethylammonium propane (DOTAP), but is not limited thereto.

In the present invention, DOTAP (dioleoyl-3-trimethylammonium propane), which is an example of a preferred cationic lipid, is a cationic emulsifier having the structure of Chemical Formula 1 below and is used as a fabric softener, and recently is used as a delivery carrier for proteins, compounds, peptides, etc., including nucleic acid carriers that form liposomes or lipid nanoparticles.

[Chemical Formula 1]

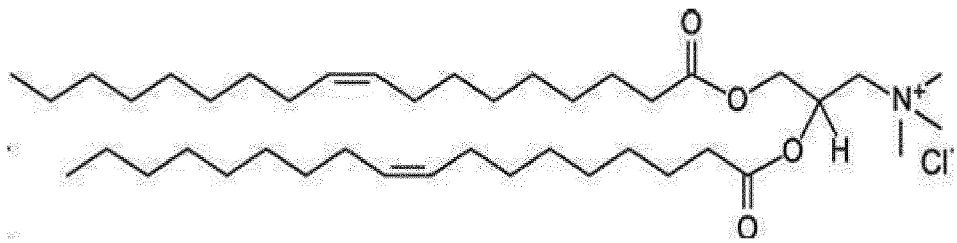

In the present invention, the composition for delivering mRNA containing a modified nucleotide including liposomes based on a cationic lipid may further include a neutral lipid.

In the present invention, the neutral lipid includes a lipid that is continuously neutral without the influence of pH change or an ionic lipid that is converted to be neutral by pH change.

In the present invention, the neutral lipid may be selected from the group consisting of 1,2-dioleoyl-sn-glycero-3-phosphoethanolamine (DOPE), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphoethanolamine (PE), phosphatidylglycerol (PG), phosphoric acid (PA), phosphatidylcholine (PC), 1,2-dioleoyl-sn-glycero-3-phospho-(1'-myo-inositol) (DOPI), and 1,2-distearoyl-sn-glycero-3-phosphoinositol (DSPI), but is not limited thereto, and is most preferably 1,2-dioleoyl-sn-glycero-3-phosphoethanolamine (DOPE), but is not limited thereto.

In the present invention, also, DOPE (1,2-dioleoyl-sn-glycero-3-phosphoethanolamine), which is an example of a preferred neutral lipid, has the structure of Chemical Formula 2 below and is used as an auxiliary lipid for forming cationic liposomes or lipid nanoparticles.

[Chemical Formula 2]

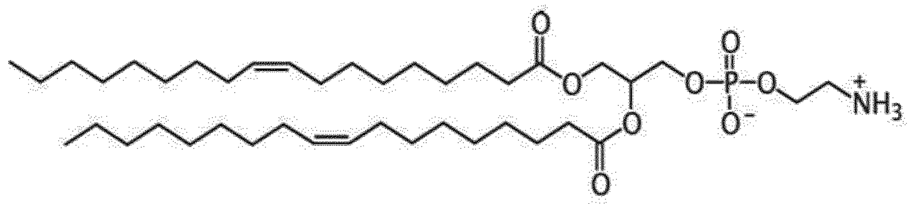

The liposomes according to the present invention may further include at least one delivery factor selected from the group consisting of protamine, albumin, transferrin, PTD (protein transduction domain), CPP (cell-penetrating peptide), PEG (polyethylene glycol), pegylated lipid, metal ion-linked lipid, and macrophage targeting moiety.
In the present invention, the weight ratio of cationic lipid to neutral lipid is 1:9 to 9.5:0.5, preferably 2:8 to 9:1, more preferably 3:7 to 8:2, most preferably 4:6 to 7:3, but is not limited thereto.
If the weight ratio thereof falls out of the above range, mRNA delivery efficiency may be greatly reduced.
In the present invention, the composition for delivering mRNA containing a modified nucleotide including liposomes based on a cationic lipid may further include cholesterol.
In the liposomes or lipid nanoparticles of the present invention, the weight ratio of cationic lipid to cholesterol is 6:1 to 1:3, preferably 4:1 to 1.0:2.5, more preferably 3:1 to 1:2, most preferably 2.5:1.0 to 1.0:1.5, but is not limited thereto.
Moreover, in the present invention, when the cationic lipid, neutral lipid, and cholesterol according to the present invention are all included, the weight ratio of cationic lipid to neutral lipid to cholesterol is 1.0-9.5:0.5-9.0:0.05-3.00, preferably 3-8:7-1:0.45-7.00, more preferably 1.0-3.5:1.0-3.5:0.5-3.0, but is not limited thereto.
If the weight ratio thereof falls out of the above range, mRNA delivery efficiency may be greatly reduced.
In an embodiment of the present invention, the weight ratio of cationic lipid to neutral lipid to cholesterol is set to 2:2:1 (40:40:20, w/w/w), but is not limited thereto.
Upon additional inclusion of cholesterol, for example, when DOTAP and DOPE are used in a weight ratio of 1:1, cholesterol may be mixed in a weight ratio of 0.20-0.85, preferably 0.4-0.6 relative to DOTAP to form liposomes.
Also, in the composition for delivering mRNA containing a modified nucleotide including liposomes based on a cationic lipid according to the present invention, the mixing ratio of liposomes and mRNA may be represented as N:P ratio, and the N:P ratio affects mRNA expression and stability of the composition.
In the present invention, the N:P ratio of liposomes or lipid nanoparticles and mRNA may be 0.2:1.0 to 1.4:1.0, preferably 0.23:1.00 to 1.0:1.0, more preferably 0.46:1.00 to 1.0:1.0, but is not limited thereto. In an exemplary embodiment of the present invention, the N:P ratio at 0.6:1.0 is used.
In the present invention, the composition for delivering mRNA containing a modified nucleotide including liposomes based on a cationic lipid may further include an immune enhancer, but the use of an immune enhancer is not essential, and a sufficient vaccine effect is exhibited even in the absence of an immune enhancer. The immune enhancer usable in the present invention is an immune enhancer selected from the group consisting of a material responding to a pattern recognition receptor (PRR) corresponding to a pathogen-associated molecular pattern (PAMP), CpG DNA, lipoprotein, flagella, poly I:C, saponin, squalene, tricaprin, 3D-MPL, and detoxified lipooligosaccharide (dLOS), but is not limited thereto.
In the immune enhancer, the detoxified lipooligosaccharide (dLOS) may be a material disclosed in Korean Patent No. 1509456 or Korean Patent No. 2042993, but the present invention is not limited thereto.
In the present invention, mRNA is capable of encoding a peptide or protein that may act as an immunogen.
Here, mRNA may be mRNA having at least one open reading frame (ORF) that may be translated by a cell or organism, which is typically provided along with mRNA. The product of this translation is an antigen, preferably a peptide or protein acting as an immunogen. Such a product may also be a fusion protein composed of two or more immunogens, for example, a fusion protein composed of two or more epitopes, peptides, or proteins derived from the same or different viral proteins, in which the epitopes, peptides, or proteins may be connected by a linker sequence.
Also, mRNA may be understood to be synthetic mRNA, namely an mRNA molecule that does not occur naturally. A synthetic mRNA molecule may be understood as a non-natural mRNA molecule. Such an mRNA molecule may be non-natural due to (non-naturally occurring) individual sequences and/or other non-naturally occurring alterations, such as structural alterations of nucleotides. A synthetic mRNA molecule may be designed and/or produced by genetic engineering methods corresponding to a desired synthetic sequence (heterologous sequence) of nucleotide.
Furthermore, mRNA may exhibit modifications that increase resistance to degradation *in vivo* (e.g. degradation by exo- or endo-nucleases) and/or degradation *in vitro* (e.g. by the preparation process before administration of a vaccine, for example, in the process of preparing a vaccine solution to be administered). Stabilization of RNA may be achieved,

for example, by providing a 5'-CAP structure, poly-A-tail, or any other UTR modification. Stabilization of RNA may also be achieved by chemical modification or modification of G/C content of nucleic acids. A variety of other methods are known in the art and are applicable to the present invention.

The composition for delivering mRNA containing a modified nucleotide including liposomes based on a cationic lipid according to the present invention may be in the form in which mRNA containing a modified nucleotide is encapsulated inside liposomes or in which mRNA containing a modified nucleotide additionally binds to the outside as well as inside the liposomes.

Also, the composition of the present invention may be characterized by having a multi-lamellar vesicle (MLV) structure, but is not limited thereto.

Also, the composition of the present invention may be characterized in that the surface charge value is a negative value, but is not limited thereto.

Another aspect of the present invention pertains to a pharmaceutical composition for preventing or treating a disease selected from the group consisting of cancer, a tumor, an autoimmune disease, a genetic disease, an inflammatory disease, a viral infection, and a bacterial infection, including the composition for delivering mRNA containing a modified nucleotide described above as an active ingredient.

In the present invention, "prevention" refers to any action that inhibits the above-mentioned disease or delays the progression thereof by administration of the pharmaceutical composition according to the present invention.

In the present invention, "treatment" refers to any action that ameliorates or advantageously changes symptoms of the above-mentioned disease by administration of the pharmaceutical composition according to the present invention.

The pharmaceutical composition of the present invention may be included in combination with at least one pharmaceutically or physiologically acceptable carrier, diluent, or excipient. The pharmaceutical composition may include buffers such as neutral buffered saline, phosphate buffered saline, citrate buffer, etc.; carbohydrates such as glucose, mannose, sucrose, dextran, or mannitol; proteins; polypeptides or amino acids such as glycine; antioxidants; chelating agents such as EDTA or glutathione; adjuvants (e.g. aluminum hydroxide); and preservatives.

The pharmaceutical composition of the present invention may be administered orally or parenterally, for example through intravenous administration, subcutaneous administration, intradermal administration, intramuscular administration, intraperitoneal administration, intratumoral administration, intracerebral administration, intracranial administration, intrapulmonary administration, and intrarectal administration, but the present invention is not limited thereto.

The pharmaceutical composition of the present invention is administered in a pharmaceutically effective amount.

In the present invention, a "pharmaceutically effective amount" is an amount sufficient to treat a disease at a reasonable benefit/risk ratio applicable to medical treatment, and the effective amount may be determined depending on the type of disease of a patient, severity of disease, drug activity, drug sensitivity, time of administration, route of administration, excretion rate, duration of treatment, type of concomitant drug, and other factors well known in the medical field.

The pharmaceutical composition of the present invention may be administered as an individual therapeutic agent or in combination with other therapeutic agents, may be administered sequentially or simultaneously with a conventional therapeutic agent, and may be administered once or multiple times. Taking into consideration all of the above factors, it is important to administer an amount that may obtain the maximum effect in the minimum amount without side effects, which may be easily determined by those skilled in the art.

Specifically, the effective amount of the pharmaceutical composition of the present invention may vary depending on the patient's age, gender, condition, body weight, absorption rate of the active ingredient in the body, inactivation rate, and excretion rate, type of disease, and type of concomitant drug.

Since the pharmaceutical composition of the present invention includes the composition for delivering mRNA containing a modified nucleotide described above as an active ingredient, a redundant description thereof is omitted to avoid excessive complexity in the present specification.

Still another aspect of the present invention pertains to a method of preventing or treating cancer, a tumor, an autoimmune disease, an inflammatory disease, a viral infection, or a bacterial infection, including administering the composition for delivering mRNA containing a modified nucleotide according to the present invention to a patient in need of prevention or treatment for cancer, a tumor, an autoimmune disease, an inflammatory disease, a viral infection, or a bacterial infection.

The amount of mRNA that is administered may be 1 to 5 pg, 5 to 10 pg, 10 to 15 pg, 15 to 20 pg, 10 to 25 pg, 20 to 25 pg, 20 to 50 pg, 30 to 50 pg, 40 to 50 pg, 40 to 60 pg, 60 to 80 pg, 60 to 100 pg, 50 to 100 pg, 80 to 120 pg, 40 to 120 pg, 40 to 150 pg, 50 to 150 μg, 50 to 200 μg, 80 to 200 μg, 100 to 200 μg, 120 to 250 pg, 150 to 250 pg, 180 to 280 pg, 200 to 300 pg, 50 to 300 pg, 80 to 300 μg, 100 to 300 μg, 40 to 300 μg, 50 to 350 μg, 100 to 350 pg, 200 to 350 pg, 300 to 350 pg, 320 to 400 pg, 40 to 380 pg, 40 to 100 pg, 100 to 400 pg, 200 to 400 pg, 300 to 400 pg, 30 to 500 pg, 40 to 500 pg, 50 to 500 pg, 100 to 500 pg, 200 to 500 pg, 300 to 500 pg, 400 to 500 pg, 40 to 600 pg, 100 to 600 pg, 200 to 600 pg, 400 to 600 pg, 40 to 700 pg, 100 to 700 pg, 300 to 700 pg, 500 to 700 pg, 40 to 800 pg, 100 to 800 pg, 200 to 800 pg, 400 to 800 pg, or 600 to 800 μg per dose, but is not limited thereto.

Yet another aspect of the present invention pertains to a vaccine including the composition for delivering mRNA containing a modified nucleotide as an active ingredient.

In the present invention, the vaccine may be a vaccine against viruses capable of infecting humans and animals, such as influenza, coronavirus, shingles, human papillomavirus, Zika virus, herpes virus, AIDS virus, SFTS virus, measles virus, varicella virus, Ebola virus, MERS virus, hepatitis virus, avian influenza, rabies virus, and foot-and-mouth disease virus, but the present invention is not limited thereto.

In the present invention, the vaccine may be a multivalent vaccine, and the virus included in the multivalent vaccine may include two or more viruses selected from the group consisting of viruses capable of infecting humans and animals, such as influenza, coronavirus, shingles, human papillomavirus, Zika virus, herpes virus, AIDS virus, SFTS virus, measles virus, varicella virus, Ebola virus, MERS virus, hepatitis virus, avian influenza, rabies virus, and foot-and-mouth disease virus, but the present invention is not limited thereto.

In the present invention, the vaccine includes mRNA containing at least one modified nucleotide having an ORF encoding at least one viral antigen polypeptide or an immunogenic fragment thereof.

In the present invention, the vaccine may include the composition for delivering mRNA containing a modified nucleotide at an appropriate concentration in consideration of the body weight, age, dietary stage, and/or immunity of an administration subject within a range of the purpose of preventing a disease caused by a peptide or protein that may act as an immunogen by the aforementioned mRNA.

In the present invention, the vaccine may further include at least one selected from the group consisting of a carrier, a diluent, an excipient, and an adjuvant. The type of carrier is not particularly limited, but may include any and all solvents, dispersion media, coatings, stabilizers, preservatives, antibacterial and antifungal agents, isotonic agents, absorption delaying agents, and the like.

In the present invention, the vaccine may be administered through oral, parenteral, subcutaneous, intramuscular, intradermal, sublingual, transdermal, intrarectal, transmucosal, surface area via inhalation, or buccal routes, or combinations thereof.

In the present invention, the vaccine may be administered once or several times, and also intermittently, for example daily for days, weeks, or months, in the same amount or in different amounts, depending on the desired duration and effectiveness of vaccination or treatment. An injectable solution thereof may be administered by injection in a desired amount or by spraying subcutaneously or intranasally, or alternatively by continuous infusion.

In the present invention, the vaccine may be provided in the form of a lyophilized formulation, but is not limited thereto.

Since the vaccine of the present invention includes the composition for delivering mRNA containing a modified nucleotide described above as an active ingredient, a redundant description thereof is omitted to avoid excessive complexity in the present specification.

Still yet another aspect of the present invention pertains to a functional cosmetic composition including the composition for delivering mRNA containing a modified nucleotide described above as an active ingredient.

The cosmetic composition according to the present invention may include components commonly used in cosmetic compositions, for example, typical adjuvants such as sequestering agents, active ingredients (e.g. sodium hyaluronate, tocopheryl acetate, etc.), preservatives, thickeners, and fragrances, and carriers.

Moreover, the cosmetic composition according to the present invention may be prepared in the form of a typical formulation in the art, for example, an emulsion formulation or a solubilization formulation. The emulsion formulation may be exemplified by nutrient lotion, cream, essence, etc., and the solubilization formulation may be exemplified by softening lotion. Also, the cosmetic composition of the present invention may be prepared in the form of an adjuvant that may be applied topically or systemically, which is commonly used in the field of dermatology, by containing a dermatologically acceptable medium or base in addition to cosmetics.

Examples of suitable cosmetic formulations may include solutions, gels, solid or anhydrous paste products, oil-in-water emulsions, suspensions, microemulsions, microcapsules, microgranules, ionic (liposomes) or nonionic follicular dispersions, creams, toners, lotions, powders, ointments, sprays, and conceal sticks. Also, the cosmetic composition of the present invention may be prepared in the form of foam or an aerosol composition further containing a compressed propellant.

In addition, the cosmetic composition of the present invention may further include a fatty material, an organic solvent, a solubilizing agent, a thickening agent, a gelling agent, a softening agent, an antioxidant, a suspending agent, a stabilizer, a foaming agent, a fragrance, a surfactant, water, an ionic or nonionic emulsifier, a filler, a sequestering agent, a chelating agent, a preservative, a vitamin, a blocking agent, a humectant, an essential oil, a dye, a pigment, a hydrophilic or lipophilic activating agent, a lipid vesicle, or an adjuvant commonly used in cosmetology or dermatology, such as any other ingredient commonly used in cosmetics. Moreover, the above ingredients may be introduced in amounts generally used in the field of dermatology.

Examples of products to which the cosmetic composition of the present invention may be added may include cosmetics such as astringent lotions, softening lotions, nutrient lotions, various creams, essences, packs, foundations,

etc., as well as cosmetics such as cleansers, face washes, soaps, treatments, beauty essences, etc.

Specific formulations of the cosmetic composition of the present invention include skin lotion, skin softener, skin toner, astringent, lotion, milk lotion, moisture lotion, nutrient lotion, massage cream, nutrient cream, moisture cream, hand cream, essence, nutrient essence, pack, soap, shampoo, cleansing foam, cleansing lotion, cleansing cream, body lotion, body cleanser, emulsion, pressed powder, loose powder, patch, spray, and the like.

Since the cosmetic composition of the present invention includes the composition for delivering mRNA containing a modified nucleotide described above as an active ingredient, a redundant description thereof is omitted to avoid excessive complexity in the present specification.

A further aspect of the present invention pertains to the use of the composition for delivering mRNA containing a modified nucleotide including liposomes based on a cationic lipid for preventing or treating a disease selected from the group consisting of cancer, a tumor, an autoimmune disease, a genetic disease, an inflammatory disease, a viral infection, and a bacterial infection.

Still a further aspect of the present invention pertains to the use of the composition for delivering mRNA containing a modified nucleotide including liposomes based on a cationic lipid for the manufacture of a medicament for preventing or treating a disease selected from the group consisting of cancer, a tumor, an autoimmune disease, a genetic disease, an inflammatory disease, a viral infection, and a bacterial infection.

## Examples

A better understanding of the present invention may be obtained through the following examples. These examples are merely set forth to illustrate the present invention, and are not to be construed as limiting the scope of the present invention, as will be obvious to those skilled in the art.

### Example 1: Preparation of IVT mRNA containing modified nucleotide

Modified nucleotides used were N1-methylpseudouridine (m1Ψ, TriLink BioTechnologies, USA) and 5-methoxyuridine (5moU, TriLink BioTechnologies, USA), and target mRNA templates used were Firefly Luciferase (F. Luc) and SARS-CoV-2 omicron spike protein mRNA (EG-COVARo).

mRNA containing a modified nucleotide was synthesized by substituting any uridine with the corresponding modified nucleotide. Here, specific examples of synthesized mRNA are as follows, and the ORFs of Luciferase and SARS-CoV-2 omicron spike protein mRNA (EG-COVARo) used and the amino acid sequences constructed thereby are shown in Table 1 below.

1) Unmodified Firefly Luciferase mRNA

2) 5moU modified Firefly Luciferase mRNA

3) m1Ψ modified Firefly Luciferase mRNA

4) Unmodified SARS-CoV-2 omicron spike protein mRNA

5) 5moU modified SARS-CoV-2 omicron spike protein mRNA

6) m1Ψ modified SARS-CoV-2 omicron spike protien mRNA

[Table 1]

| SEQ ID NO: | Name | Sequence |
|---|---|---|
| 1 | Firefly Lucifera se ORF | AUGACCAGCAAGGUGUACGACCCCGAGCAGCGGAAGCGGAUGA<br>UCACCGGCCCCCAGUGGUGGGCCCGGUGCAAGCAGAUGAACGU<br>GCUGGACAGCUUCAUCAACUACUACGACAGCGAGAAGCACGCC<br>GAGAACGCCGUGAUCUUCCUGCACGGCAACGCCGCCAGCAGCU<br>ACCUGUGGCGGCACGUGGUGCCCCACAUCGAGCCCGUGGCCCG<br>GUGCAUCAUCCCCGACCUGAUCGGCAUGGGCAAGAGCGGCAAG<br>AGCGGCAACGGCAGCUACCGGCUGCUGGACCACUACAAGUACC<br>UGACCGCCUGGUUCGAGCUGCUGAACCUGCCCAAGAAGAUCAU<br>CUUCGUGGGCCACGACUGGGGCGCCUGCCUGGCCUUCCACUAC<br>AGCUACGAGCACCAGGACAAGAUCAAGGCCAUCGUGCACGCCG<br>AGAGCGUGGUGGACGUGAUCGAGAGCUGGGACGAGUGGCCCGA<br>CAUCGAGGAGGACAUCGCCCUGAUCAAGAGCGAGGAGGGCGAG<br>AAGAUGGUGCUGGAGAACAACUUCUUCGUGGAGACCAUGCUGC<br>CCAGCAAGAUCAUGCGGAAGCUGGAGCCCGAGGAGUUCGCCGC<br>CUACCUGGAGCCCUUCAAGGAGAAGGGCGAGGUGCGGCGGCCC<br>ACCCUGAGCUGGCCCCGGGAGAUCCCCCUGGUGAAGGGCGGCA<br>AGCCCGACGUGGUGCAGAUCGUGCGGAACUACAACGCCUACCU<br>GCGGGCCAGCGACGACCUGCCCAAGAUGUUCAUCGAGAGCGAC<br>CCCGGCUUCUUCAGCAACGCCAUCGUGGAGGGCGCCAAGAAGU<br>UCCCCAACACCGAGUUCGUGAAGGUGAAGGGCCUGCACUUCAG<br>CCAGGAGGACGCCCCCGACGAGAUGGGCAAGUACAUCAAGAGC<br>UUCGUGGAGCGGGUGCUGAAGAACGAGCAGUGA |
| 2 | Firefly Lucifera<br><br>se Amino acid | MTSKVYDPEQRKRMITGPQWWARCKQMNVLDSFINYYDSEKHA<br>ENAVIFLHGNAASSYLWRHVVPHIEPVARCIIPDLIGMGKSGK<br>SGNGSYRLLDHYKYLTAWFELLNLPKKIIFVGHDWGACLAFHY<br>SYEHQDKIKAIVHAESVVDVIESWDEWPDIEEDIALIKSEEGE<br>KMVLENNFFVETMLPSKIMRKLEPEEFAAYLEPFKEKGEVRRP<br>TLSWPREIPLVKGGKPDVVQIVRNYNAYLRASDDLPKMFIESD<br>PGFFSNAIVEGAKKFPNTEFVKVKGLHFSQEDAPDEMGKYIKS<br>FVERVLKNEQ |

(continued)

| SEQ ID NO: | Name | Sequence |
|---|---|---|
| 3 | SARS-CoV-2 omicron spike protein ORF | AUGUUCGUGUUCCUGGUGCUGCUGCCCCUGGUGAGCAGCCAGU<br>GCGUGAACCUGACCACCAGAACCCAGCUGCCCCCCGCCUACAC<br>CAACAGCUUCACCAGAGGCGUGUACUACCCCGACAAGGUGUUC<br>AGAAGCAGCGUGCUGCACAGCACCCAGGACCUGUUCCUGCCCU<br>UCUUCAGCAACGUGACCUGGUUCCACGUGAUCAGCGGCACCAA<br>CGGCACCAAGAGAUUCGACAACCCCGUGCUGCCCUUCAACGAC<br>GGCGUGUACUUCGCCAGCAUCGAGAAGAGCAACAUCAUCAGAG<br>GCUGGAUCUUCGGCACCACCCUGGACAGCAAGACCCAGAGCCU<br>GCUGAUCGUGAACAACGCCACCAACGUGGUGAUCAAGGUGUGC<br>GAGUUCCAGUUCUGCAACGACCCCUUCCUGGACCACAAGAACA<br>ACAAGAGCUGGAUGGAGAGCGAGUUCAGAGUGUACAGCAGCGC<br>CAACAACUGCACCUUCGAGUACGUGAGCCAGCCCUUCCUGAUG<br>GACCUGGAGGGCAAGCAGGGCAACUUCAAGAACCUGAGAGAGU<br>UCGUGUUCAAGAACAUCGACGGCUACUUCAAGAUCUACAGCAA<br>GCACACCCCCAUCAUCGUGAGAGAGCCCGAGGACCUGCCCCAG<br>GGCUUCAGCGCCCUGGAGCCCCUGGUGGACCUGCCCAUCGGCA<br>UCAACAUCACCAGAUUCCAGACCCUGCUGGCCCUGCACAGAAG<br>CUACCUGACCCCCGGCGACAGCAGCAGCGGCUGGACCGCCGGC<br>GCCGCCGCCUACUACGUGGGCUACCUGCAGCCCAGAACCUUCC<br>UGCUGAAGUACAACGAGAACGGCACCAUCACCGACGCCGUGGA<br>CUGCGCCCUGGACCCCCUGAGCGAGACCAAGUGCACCCUGAAG<br>AGCUUCACCGUGGAGAAGGGCAUCUACCAGACCAGCAACUUCA<br>GAGUGCAGCCCACCGAGAGCAUCGUGAGAUUCCCCAACAUCAC<br>CAACCUGUGCCCCUUCGACGAGGUGUUCAACGCCACCAGAUUC<br>GCCAGCGUGUACGCCUGGAACAGAAAGAGAAUCAGCAACUGCG<br>UGGCCGACUACAGCGUGCUGUACAACCUGGCCCCCUUCUUCAC<br>CUUCAAGUGCUACGGCGUGAGCCCCACCAAGCUGAACGACCUG<br>UGCUUCACCAACGUGUACGCCGACAGCUUCGUGAUCAGAGGCG<br>ACGAGGUGAGACAGAUCGCCCCCGGCCAGACCGGCAACAUCGC<br>CGACUACAACUACAAGCUGCCCGACGACUUCACCGGCUGCGUG<br>AUCGCCUGGAACAGCAACAAGCUGGACAGCAAGGUGAGCGGCA<br>ACUACAACUACCUGUACAGACUGUUCAGAAAGAGCAACCUGAA<br>GCCCUUCGAGAGAGACAUCAGCACCGAGAUCUACCAGGCCGGC<br>AACAAGCCCUGCAACGGCGUGGCCGGCUUCAACUGCUACUUCC<br>CCCUGAAGAGCUACAGCUUCAGACCCACCUACGGCGUGGGCCA<br>CCAGCCCUACAGAGUGGUGGUGCUGAGCUUCGAGCUGCUGCAC<br>GCCCCCGCCACCGUGUGCGGCCCCAAGAAGAGCACCAACCUGG<br>UGAAGAACAAGUGCGUGAACUUCAACUUCAACGGCCUGAAGGG<br>CACCGGCGUGCUGACCGAGAGCAACAAGAAGUUCCUGCCCUUC<br>CAGCAGUUCGGCAGAGACAUCGCCGACACCACCGACGCCGUGA<br>GAGACCCCCAGACCCUGGAGAUCCUGGACAUCACCCCCUGCAG<br>CUUCGGCGGCGUGAGCGUGAUCACCCCCGGCACCAACACCAGC<br>AACCAGGUGGCCGUGCUGUACCAGGGCGUGAACUGCACCGAGG<br>UGCCCGUGGCCAUCCACGCCGACCAGCUGACCCCCACCUGGAG<br>AGUGUACAGCACCGGCAGCAACGUGUUCCAGACCAGAGCCGGC<br>UGCCUGAUCGGCGCCGAGUACGUGAACAACAGCUACGAGUGCG |

(continued)

| SEQ ID NO: | Name | Sequence |
|---|---|---|
| | | ACAUCCCCAUCGGCGCCGGCAUCUGCGCCAGCUACCAGACCCA<br>GACCAAGAGCCACCAGCAGGCCCAGAGCGUGGCCAGCCAGAGC<br>AUCAUCGCCUACACCAUGAGCCUGGGCGCCGAGAACAGCGUGG<br>CCUACAGCAACAACAGCAUCGCCAUCCCCACCAACUUCACCAU<br>CAGCGUGACCACCGAGAUCCUGCCCGUGAGCAUGACCAAGACC<br>AGCGUGGACUGCACCAUGUACAUCUGCGGCGACAGCACCGAGU<br>GCAGCAACCUGCUGCUGCAGUACGGCAGCUUCUGCACCCAGCU<br>GAAGAGAGCCCUGACCGGCAUCGCCGUGGAGCAGGACAAGAAC<br>ACCCAGGAGGUGUUCGCCCAGGUGAAGCAGAUCUACAAGACCC<br>CCCCCAUCAAGUACUUCGGCGGCUUCAACUUCAGCCAGAUCCU<br>GCCCGACCCCAGCAAGCCCAGCAAGAGAAGCUUCAUCGAGGAC<br>CUGCUGUUCAACAAGGUGACCCUGGCCGACGCCGGCUUCAUCA<br>AGCAGUACGGCGACUGCCUGGGCGACAUCGCCGCCAGAGACCU<br>GAUCUGCGCCCAGAAGUUCAAGGGCCUGACCGUGCUGCCCCCC<br>CUGCUGACCGACGAGAUGAUCGCCCAGUACACCAGCGCCCUGC<br>UGGCCGGCACCAUCACCAGCGGCUGGACCUUCGGCGCCGGCGC<br>CGCCCUGCAGAUCCCCUUCGCCAUGCAGAUGGCCUACAGAUUC<br>AACGGCAUCGGCGUGACCCAGAACGUGCUGUACGAGAACCAGA<br>AGCUGAUCGCCAACCAGUUCAACAGCGCCAUCGGCAAGAUCCA<br>GGACAGCCUGAGCAGCACCGCCAGCGCCCUGGGCAAGCUGCAG<br>GACGUGGUGAACCACAACGCCCAGGCCCUGAACACCCUGGUGA<br>AGCAGCUGAGCAGCAAGUUCGGCGCCAUCAGCAGCGUGCUGAA<br>CGACAUCUUCAGCAGACUGGACCCCCCCGAGGCCGAGGUGCAG<br>AUCGACAGACUGAUCACCGGCAGACUGCAGAGCCUGCAGACCU<br>ACGUGACCCAGCAGCUGAUCAGAGCCGCCGAGAUCAGAGCCAG<br>CGCCAACCUGGCCGCCACCAAGAUGAGCGAGUGCGUGCUGGGC<br>CAGAGCAAGAGAGUGGACUUCUGCGGCAAGGGCUACCACCUGA<br>UGAGCUUCCCCCAGAGCGCCCCCCACGGCGUGGUGUUCCUGCA<br>CGUGACCUACGUGCCCGCCCAGGAGAAGAACUUCACCACCGCC<br>CCCGCCAUCUGCCACGACGGCAAGGCCCACUUCCCCAGAGAGG<br>GCGUGUUCGUGAGCAACGGCACCCACUGGUUCGUGACCCAGAG<br>AAACUUCUACGAGCCCCAGAUCAUCACCACCGACAACACCUUC<br>GUGAGCGGCAACUGCGACGUGGUGAUCGGCAUCGUGAACAACA<br>CCGUGUACGACCCCCUGCAGCCCGAGCUGGACAGCUUCAAGGA<br>GGAGCUGGACAAGUACUUCAAGAACCACACCAGCCCCGACGUG<br>GACCUGGGCGACAUCAGCGGCAUCAACGCCAGCGUGGUGAACA<br>UCCAGAAGGAGAUCGACAGACUGAACGAGGUGGCCAAGAACCU<br>GAACGAGAGCCUGAUCGACCUGCAGGAGCUGGGCAAGUACGAG<br>CAGUACAUCAAGUGGCCCUGGUACAUCUGGCUGGGCUUCAUCG<br>CCGGCCUGAUCGCCAUCGUGAUGGUGACCAUCAUGCUGUGCUG<br>CAUGACCAGCUGCUGCAGCUGCCUGAAGGGCUGCUGCAGCUGC<br>GGCAGCUGCUGCAAGUUCGACGAGGACGACAGCGAGCCCGUGC<br>UGAAGGGCGUGAAGCUGCACUACACCUGA |

(continued)

| SEQ ID NO: | Name | Sequence |
|---|---|---|
| 4 | SARS-CoV-2 omicron spike protein Amino Acid | MFVFLVLLPLVSSQCVNLTTRTQLPPAYTNSFTRGVYYPDKVF<br>RSSVLHSTQDLFLPFFSNVTWFHVISGTNGTKRFDNPVLPFND<br>GVYFASIEKSNIIRGWIFGTTLDSKTQSLLIVNNATNVVIKVC<br>EFQFCNDPFLDHKNNKSWMESEFRVYSSANNCTFEYVSQPFLM<br>DLEGKQGNFKNLREFVFKNIDGYFKIYSKHTPIIVREPEDLPQ<br>GFSALEPLVDLPIGINITRFQTLLALHRSYLTPGDSSSGWTAG<br>AAAYYVGYLQPRTFLLKYNENGTITDAVDCALDPLSETKCTLK<br>SFTVEKGIYQTSNFRVQPTESIVRFPNITNLCPFDEVFNATRF<br>ASVYAWNRKRISNCVADYSVLYNLAPFFTFKCYGVSPTKLNDL<br>CFTNVYADSFVIRGDEVRQIAPGQTGNIADYNYKLPDDFTGCV<br>IAWNSNKLDSKVSGNYNYLYRLFRKSNLKPFERDISTEIYQAG<br>NKPCNGVAGFNCYFPLKSYSFRPTYGVGHQPYRVVVLSFELLH<br>APATVCGPKKSTNLVKNKCVNFNFNGLKGTGVLTESNKKFLPF<br>QQFGRDIADTTDAVRDPQTLEILDITPCSFGGVSVITPGTNTS<br>NQVAVLYQGVNCTEVPVAIHADQLTPTWRVYSTGSNVFQTRAG<br>CLIGAEYVNNSYECDIPIGAGICASYQTQTKSHQQAQSVASQS<br>IIAYTMSLGAENSVAYSNNSIAIPTNFTISVTTEILPVSMTKT<br>SVDCTMYICGDSTECSNLLLQYGSFCTQLKRALTGIAVEQDKN<br>TQEVFAQVKQIYKTPPIKYFGGFNFSQILPDPSKPSKRSFIED<br>LLFNKVTLADAGFIKQYGDCLGDIAARDLICAQKFKGLTVLPP<br>LLTDEMIAQYTSALLAGTITSGWTFGAGAALQIPFAMQMAYRF<br>NGIGVTQNVLYENQKLIANQFNSAIGKIQDSLSSTASALGKLQ<br>DVVNHNAQALNTLVKQLSSKFGAISSVLNDIFSRLDPPEAEVQ<br>IDRLITGRLQSLQTYVTQQLIRAAEIRASANLAATKMSECVLG<br>QSKRVDFCGKGYHLMSFPQSAPHGVVFLHVTYVPAQEKNFTTA<br>PAICHDGKAHFPREGVFVSNGTHWFVTQRNFYEPQIITTDNTF<br>VSGNCDVVIGIVNNTVYDPLQPELDSFKEELDKYFKNHTSPDV<br>DLGDISGINASVVNIQKEIDRLNEVAKNLNESLIDLQELGKYE<br>QYIKWPWYIWLGFIAGLIAIVMVTIMLCCMTSCCSCLKGCCSC<br>GSCCKFDEDDSEPVLKGVKLHYT |

## Example 2: Preparation of liposome delivery carrier using film method

Each of DOTAP (CH2900014, Merck, Germany), DOPE (LP-R4-069, Corden Pharma, Germany), and cholesterol (W004591, Sigma-Aldrich, USA) was mixed with chloroform in a ratio of 40:40:20 and completely dissolved at 37°C for 10 minutes to form liquid solutions.

A lipid mixture was prepared by mixing the liquid solutions at a predetermined weight ratio in a round bottom flask, and the lipid mixture containing DOTAP was volatilized at 60°C for 30 minutes in a rotary evaporator (B491_R200, Buchi, Switzerland) to blow off chloroform, and a lipid membrane film was formed on the wall of the flask.

20 mM HEPES buffer containing 4% sucrose was placed in the flask and the lipid membrane was dissolved at 60°C to form liposomes. The liposomes thus formed were measured for particle size, zeta potential, and dispersity using a dynamic light scattering analyzer. The prepared liposomes were refrigerated (2-8°C) until testing.

## Example 3: Preparation of mRNA-liposome complex

Respective mRNA-liposome complexes were prepared by mixing the liposomes prepared in Example 2 (LP-DOTAP/DOPE/cholesterol (40:40:20)) and 6 types of mRNA in 20 mM HEPES buffer containing 4% sucrose.

Here, the liposome solution (LP-DOTAP/DOPE/cholesterol (40:40:20)) used was one obtained immediately after preparation or refrigerated for up to 2 weeks after preparation. The mRNA solution was stored at -70°C or less, and was used after thawing on ice immediately before use. 20 mM HEPES buffer containing 4% sucrose used was one prepared immediately before the experiment or refrigerated (2-8°C) after preparation the day before the experiment.

The N/P ratio, which is the mixing ratio of liposomes and mRNA, was calculated using the following equation.

N:P = mol number of DOTAP : mol number of mRNA * nucleotide number of mRNA

The N/P ratio of cationic liposomes and each mRNA was 0.6:1.0.

**Example 4: Confirmation of mRNA expression efficiency depending on type of modified nucleotide**

In order to confirm the *in-vivo* expression efficiency depending on the type of modified nucleotide when mixed with cationic liposomes, mRNA of each of 2) and 3) prepared in Example 1 was mixed with cationic liposomes in the same manner as in Example 3 to form mRNA-liposome complexes.

100 μL of each of the prepared mRNA-liposome complexes was intramuscularly injected into the left thigh of 6-week-old female Balb/c nude mice (Orient Bio, Korea) (IM injection).

6 hours after administration, the mice were anesthetized by intraperitoneal administration of 250 mg/kg of Avertin working solution, and 20 mL of 1x PBS was added to a luciferase substrate (VivoGlo™ Luciferin, P1043, Promega, USA) to obtain a 50 mg/mL substrate working solution, which was then diluted to 15 mg/mL before administration, and 200 μL thereof per mouse was intravenously injected through the tail vein (IV injection).

Immediately after substrate administration, the mice were placed in an *in-vivo* imaging system (IVIS) (Ami HTX, Spectral Instrument, USA), images were taken by setting the exposure time to 60 seconds, and the luciferase expression level of the region of interest (ROI) was quantified.

Thereby, as shown in FIG. 1, the ROI value of the 5moU modified mRNA + liposome group was confirmed to be about 4.3 to 10.2 times higher than that of the m1Ψ modified mRNA + liposome group.

**Example 5. Confirmation of immunogenicity depending on type of modified nucleotide**

In order to confirm *in-vivo* immunogenicity depending on the type of modified nucleotide, mRNA of each of 5) and 6) prepared in Example 1 was mixed with cationic liposomes in the same manner as in Example 3 to prepare mRNA-liposome complexes.

5, 10, 20, and 40 μg of each of the mRNA-liposome complexes based on the amount of mRNA were intramuscularly injected into the left thigh of 6-week-old female Balb/c mice (Central Lab. Animal Inc., Republic of Korea) under conditions of 100 μL per mouse and 3 times at 3-week intervals (IM injection).

Two weeks after final immunization, the mice were sacrificed, serum was separated, and the end-point titer was determined by analyzing the SARS-CoV-2 omicron receptor-binding domain (RBD) protein-specific total IgG antibody titer (logic) by indirect ELISA.

5-1: Blood colletion

Two weeks after the last administration of the mRNA-liposome complex, mice were anesthetized by intraperitoneal administration of 250 mg/kg of Avertin working solution, and whole blood was collected through cardiac blood sampling. The whole blood thus collected was transferred to a microtube, allowed to stand at room temperature for 3 hours, and centrifuged at 4°C and 15,000 rpm for 10 minutes, after which the supernatant was transferred to a new microtube and serum was obtained and stored at -20°C or less until analysis.

5-2: Analysis of antibody titer

An RBD antigen (SARS-CoV-2 spike protein, MBS335825, Mybiosource, USA) was diluted to 1 ug/mL using 1x PBS, dispensed at 100 μL/well into an immunoplate, covered with a sealing film, and allowed to stand overnight in a refrigerator at 4°C. The solution in each well was removed with an ELISA washer (Hydroflexelisa, Tecan, Switzerland), followed by washing three times using wash buffer (addition of 500 μL of Tween-20 to 1 L of 1x PBS obtained by diluting 20x PBS with purified water). A reagent diluent (1% BSA in PBS, prepared by dissolving 1 g of BSA in 100 mL of PBS) was dispensed at 200 μL/well into the immunoplate, covered with a sealing film, and allowed to stand in a reactor at

37°C for 1 hour. The solution in each well was removed with an ELISA washer, followed by washing three times using wash buffer. A reagent diluent was dispensed at 100 μL/well into the immunoplate.

After diluting a negative control group (buffer administration group) at 1:50 and a vaccine administration group at 1:100 among the serum samples obtained above using a reagent diluent (1% BSA in PBS), 100 μL thereof was dispensed in line 1 of B to G of the immunoplate, and the sample was mixed by pipetting several times in the well, after which the sample was subjected to 1/2 serial dilution up to line 12 on the ELISA plate in a manner of taking 100 μL from line 1 and adding the same to line 2. As such, in order to evaluate adequacy of the test, hyperserum was diluted 1:200 using a reagent diluent, after which 100 μL thereof was dispensed in line 1 of H of the immunoplate, followed by 1/2 serial dilution in the same manner as above.

The immunoplate was covered with a sealing film, followed by reaction in a reactor at 37°C for 2 hours. The solution in each well was removed with an ELISA washer, followed by washing three times using wash buffer. A goat anti-mouse IgG antibody (Jackson Laboratory, USA) was diluted 1:5,000 using a reagent diluent, dispensed in an amount of 100 μL into the immunoplate, covered with a sealing film, and allowed to react in a reactor at 37°C for 1 hour. The solution in each well was removed with an ELISA washer, followed by washing three times using wash buffer.

A TMB substrate working solution equilibrated to room temperature was dispensed at 100 μL/well into the immunoplate and allowed to react for 12 minutes in the dark at room temperature. The reaction was stopped by dispensing a 1 N $H_2SO_4$ solution at 100 μL/well into the immunoplate, and absorbance was measured at 450 nm using an ELISA reader (Epoch, Biotek, USA).

Thereby, as shown in FIG. 2, it was confirmed that, when 5 μg or 10 μg of the complex based on the amount of mRNA was added per mouse, the binding antibody titer of the 5moU modified mRNA + liposome group was higher than that of the m1Ψ modified mRNA + liposome group.

**Example 6: Identification of inflammatory response depending on type of modified nucleotide**

In order to confirm the inflammatory response of the mRNA-liposome complex depending on the type of modified nucleotide, mRNA of each of 4), 5), and 6) prepared in Example 1 was mixed with cationic liposomes in the same manner as in Example 3 to form mRNA-liposome complexes.

Mouse immune cell line J774A.1 cells (TIB-67, ATCC, USA) were seeded at $1\times10^6$ cells/well in a 24-well cell culture plate, followed by culture overnight at 37°C in a $CO_2$ incubator. When cell confluency was 70-80%, the cells were treated with each mRNA-liposome complex at 10 μg/well based on mRNA, followed by culture overnight at 37°C in a $CO_2$ incubator. In a positive control group, the cells were treated with LPS at 10 ug/well. Thereafter, the plate was centrifuged at 3,000 rpm and 4°C for 3 minutes, after which the supernatant was decanted and each cell culture fluid was collected.

The concentration of inflammatory cytokine marker secreted in each culture fluid was measured using ELISA, and the ELISA kit used was as follows.

- IL-1β: Mouse IL-1β quantikine ELISA (SMLB00C, R&D systems, USA)
- IL-6: Mouse IL-6 quantikine ELISA (SM6000B, R&D systems, USA)
- TNF-α: Mouse TNF-α quantikine ELISA (SMTA00B, R&D systems, USA)
- IFN-α: Mouse IFN-α all subtype quantikine ELISA (MFNAS0, R&D systems, USA)

Thereby, as shown in Table 2 below, the secretion of IL-1β, IL-6, TNF-α, and IFN-α in the mRNA using unmodified nucleotide + liposome group was about 1.9 to 159.5 times higher than that in the 5moU modified mRNA + liposome group, and also, the secretion of IL-6, TNF-α, and IFN-α in the m1Ψ modified mRNA + liposome group was about 2.5 to 94.3 times higher than that in the 5moU modified mRNA + liposome group.

[Table 2]

Secretion of inflammatory cytokine depending on UTP modification upon liposome mixing (pg/mL)

| (pg/mL) | Untreated | LPS | Omicron mRNA + Liposome | | |
|---|---|---|---|---|---|
| | | | UTP | 5moU | m1Ψ |
| **IL-1β** | < 12.5 | 110.4 | 525.5 | < 12.5 | < 12.5 |
| **IL-6** | 5.5 | 33575.0 | 1994.4 | < 12.5 | 1179.0 |
| **TNF-α** | < 109 | 40221.7 | 2119.7 | 666.6 | 1706.9 |
| **IFN-α** | 65.9 | 269.5 | 124.0 | 66.8 | 1410.9 |

In general, it is known that, when IVT is performed using a modified nucleotide, the inflammatory response is

low compared to when using an unmodified nucleotide due to the influence of noninflammatory mRNA. Among modified nucleotides, the 5moU modified mRNA + liposome group showed a lower level of inflammatory cytokine secretion than the m1ψ modified mRNA + liposome group, confirming that the inflammatory response was greatly reduced in the combination of 5moU modified mRNA and liposomes.

Having described specific parts of the present invention in detail above, it will be obvious to those skilled in the art that these specific descriptions are only preferred embodiments, and the scope of the present invention is not limited thereby. Accordingly, the substantial scope of the present invention will be defined by the appended claims and equivalents thereto.

Industrial Applicability

According to the present invention, a composition for delivering mRNA containing a modified nucleotide has superior storage stability and exhibits high intracellular delivery and expression efficiencies *in vivo,* thereby increasing the stability and efficiency of mRNA vaccines for cancer treatment or mRNA vaccines for preventing viral infections and other mRNA vaccines or therapeutics.

## Claims

1. A composition for delivering mRNA containing a modified nucleotide, comprising liposomes based on a cationic lipid.

2. The composition according to claim 1, wherein the modified nucleotide is selected from the group consisting of pseudouridine (Ψ), N1-methylpseudouridine (m1Ψ), 5-methyluridine (m5U), 2-thiouridine (s2U), 2'-O-methyl-uridine (Um), 5-methylcytidine (m5C), and 5-methoxyuridine (5moU).

3. The composition according to claim 1, wherein the liposomes further comprise a neutral lipid.

4. The composition according to claim 1, wherein the liposomes further comprises cholesterol.

5. The composition according to claim 1, wherein the cationic lipid is at least one selected from the group consisting of dimethyldioctadecylammonium bromide (DDA), C12-200, 1,2-dioleoyl-3-trimethylammonium propane (DOTAP), 3β-[N-(N',N'-dimethylaminoethane)carbamoyl cholesterol (DC-Chol), 1,2-dioleoyloxy-3-dimethylammonium propane (DODAP), 1,2-di-O-octadecenyl-3-trimethylammonium propane (DOTMA), 1,2-dimyristoleoyl-sn-glycero-3-ethylphosphocholine (14:1 Ethyl PC), 1-palmitoyl-2-oleoyl-sn-glycero-3-ethylphosphocholine (16:0/18:1 Ethyl PC), 1,2-dioleoyl-sn-glycero-3-ethylphosphocholine (18:1 Ethyl PC), 1,2-distearoyl-sn-glycero-3-ethylphosphocholine (18:0 Ethyl PC), 1,2-dipalmitoyl-sn-glycero-3-ethylphosphocholine (16:0 Ethyl PC), 1,2-dimyristoyl-sn-glycero-3-ethylphosphocholine (14:0 Ethyl PC), 1,2-dilauroyl-sn-glycero-3-ethylphosphocholine (12:0 Ethyl PC), N1- [2-((1S)-1-[(3-aminopropyl)amino]-4-[di(3-aminopropyl)amino]butylcarboxamido)ethyl]-3,4-di[oleyloxy]-benzamide (MVL5), 1,2-dimyristoyl-3-dimethylammonium-propane (14:0 DAP), 1,2-dipalmitoyl-3-dimethylammonium-propane (16:0 DAP), 1,2-distearoyl-3-dimethylammonium-propane (18:0 DAP), N-(4-carboxybenzyl)-N,N-dimethyl-2,3-bis(oleoyloxy)propan-1-aminium (DOBAQ), 1,2-stearoyl-3-trimethylammonium-propane (18:0 TAP), 1,2-dipalmitoyl-3-trimethylammonium-propane (16:0 TAP), 1,2-dimyristoyl-3-trimethylammonium-propane (14:0 TAP), and N4-cholesteryl-spermine (GL67).

6. The composition according to claim 3, wherein the neutral lipid is at least one selected from the group consisting of 1,2-dimyristoyl-sn-glycero-3-phosphocholine (DMPC), 1,2-dioleoyl-sn-glycero-3-phosphocholine (DOPC), 1,2-dioleoyl-sn-glycero-3-phosphoethanolamine (DOPE), 1,2-dipalmitoyl-sn-glycero-3-phosphocholine (DPPC), 1,2-distearoyl-sn-glycero-3-phosphocholine (DSPC), 1,2-dilinoleoyl-sn-glycero-3-phosphocholine (DLPC), phosphatidylserine (PS), phosphoethanolamine (PE), phosphatidylglycerol (PG), phosphoric acid (PA), phosphatidylcholine (PC), 1,2-dioleoyl-sn-glycero-3-phospho-(1'-myo-inositol) (DOPI), and 1,2-distearoyl-sn-glycero-3-phosphoinositol (DSPI).

7. The composition according to claim 3, wherein a weight ratio of the cationic lipid to the neutral lipid is 1:9 to 9.5:0.5.

8. The composition according to claim 4, wherein a weight ratio of the cationic lipid to the cholesterol is 6:1 to 1:3.

9. The composition according to claim 8, wherein a weight ratio of the cationic lipid to the neutral lipid to the cholesterol is 1.0-9.5:0.5-9.0:0.05-3.00.

**10.** The composition according to claim 1, wherein the mRNA containing the modified nucleotide encodes a peptide or protein acting as an immunogen.

**11.** The composition according to claim 1, wherein an N:P ratio of the liposomes and the mRNA containing the modified nucleotide is 0.23:1.00 to 1.39:1.00.

**12.** The composition according to claim 1, wherein the composition further comprises an immune enhancer.

**13.** The composition according to claim 12, wherein the immune enhancer comprises at least one selected from the group consisting of RAMP, saponin, CpG DNA, lipoprotein, flagella, poly I:C, squalene, tricaprin, 3D-MPL, and detoxified lipooligosaccharide (dLOS) .

**14.** A pharmaceutical composition for preventing or treating a disease selected from the group consisting of cancer, a tumor, an autoimmune disease, a genetic disease, an inflammatory disease, a viral infection, and a bacterial infection, comprising the composition according to any one of claims 1 to 13 as an active ingredient.

**15.** A vaccine comprising the composition according to any one of claims 1 to 13 as an active ingredient.

**16.** The vaccine according to claim 15, wherein the vaccine is in a form of a lyophilized formulation.

**17.** A functional cosmetic composition comprising the composition according to any one of claims 1 to 13 as an active ingredient.

**Figure 1**

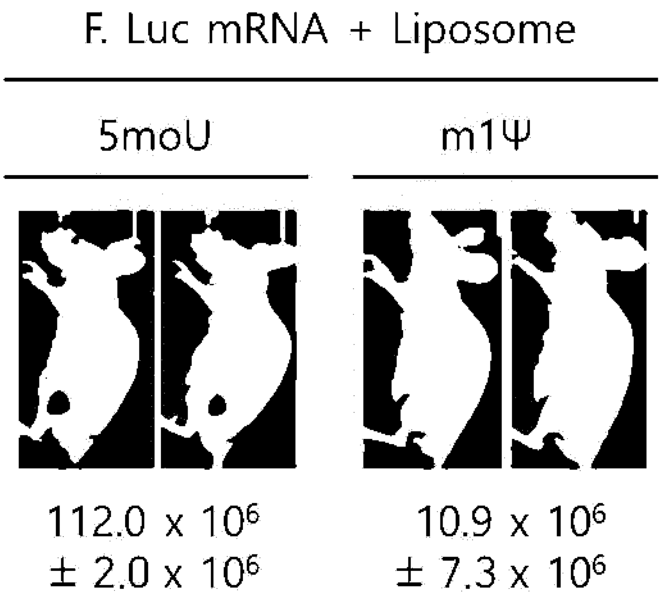
F. Luc mRNA + Liposome
5moU
m1Ψ
112.0 x $10^6$ ± 2.0 x $10^6$
10.9 x $10^6$ ± 7.3 x $10^6$

Figure 2

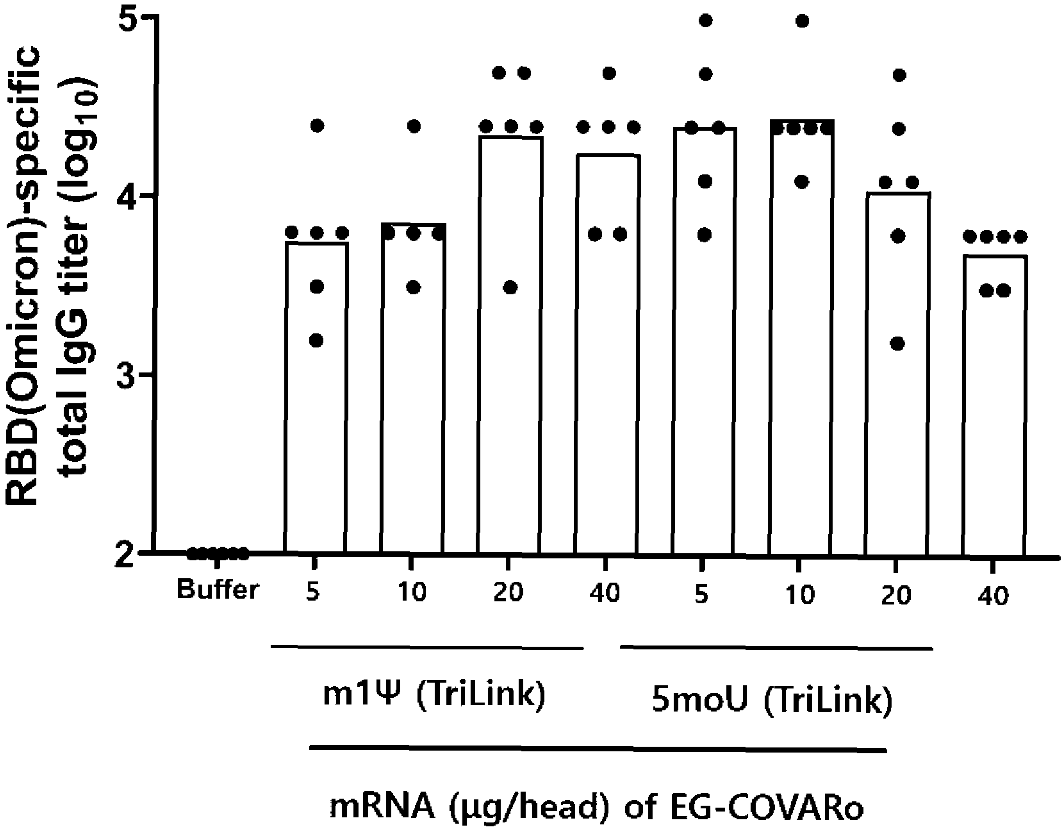
RBD(Omicron)-specific total IgG titer ($log_{10}$)
5
4
3
2
Buffer
5
10
20
40
5
10
20
40
m1Ψ (TriLink)
5moU (TriLink)
mRNA (μg/head) of EG-COVARo

**INTERNATIONAL SEARCH REPORT**

International application No.
**PCT/KR2023/013302**

**A. CLASSIFICATION OF SUBJECT MATTER**

**A61K 48/00**(2006.01)i; **A61K 9/127**(2006.01)i; **A61K 39/00**(2006.01)i; **A61P 35/00**(2006.01)i; **A61P 37/00**(2006.01)i; **A61P 29/00**(2006.01)i; **A61P 31/12**(2006.01)i; **A61P 31/04**(2006.01)i; **A61K 8/60**(2006.01)i; **A61K 8/14**(2006.01)i

According to International Patent Classification (IPC) or to both national classification and IPC

**B. FIELDS SEARCHED**

Minimum documentation searched (classification system followed by classification symbols)

A61K 48/00(2006.01); A61K 31/131(2006.01); A61K 31/7088(2006.01); A61K 31/7105(2006.01); A61K 38/17(2006.01); A61K 47/18(2006.01); A61K 47/26(2006.01); A61K 9/127(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: mRNA, 양이온성 지질(cationic lipid), 리포좀(liposome), 전달(delivery), 변형핵산(modified nucleic acid), 백신(vaccine)

**C. DOCUMENTS CONSIDERED TO BE RELEVANT**

| Category* | Citation of document, with indication, where appropriate, of the relevant passages | Relevant to claim No. |
|---|---|---|
| X | US 2017-0368200 A1 (MODERNATX, INC.) 28 December 2017 (2017-12-28)<br>See claim 1; and paragraphs [0033]-[0036], [0106], [0450], [0507], [0510], [0522], [0588], [0680], [0760] and [0819]. | 1-17 |
| X | WO 2018-160592 A1 (ARCTURUS THERAPEUTICS, INC.) 07 September 2018 (2018-09-07)<br>See paragraphs [0042], [0239]-[0249], [0327]-[0328], [0483] and [0485]. | 1,2,10,12,14-15 |
| A | KR 10-2016-0091893 A (SHIRE HUMAN GENETIC THERAPIES, INC. et al.) 03 August 2016 (2016-08-03)<br>See entire document. | 1-17 |
| A | KR 10-2128248 B1 (SHIRE HUMAN GENETIC THERAPIES, INC.) 01 July 2020 (2020-07-01)<br>See entire document. | 1-17 |
| A | KR 10-2022-0117133 A (BIOPHARMA CORP.) 23 August 2022 (2022-08-23)<br>See entire document. | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 December 2023** | **08 December 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2023/013302**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2023/013302**

| Patent document cited in search report | Publication date (day/month/year) | Patent family member(s) | Publication date (day/month/year) |
|---|---|---|---|
| US 2017-0368200 A1 | 28 December 2017 | AU 2017-213503 A1 | 31 August 2017 |
| | | AU 2017-213503 B2 | 20 June 2019 |
| | | AU 2018-200374 A1 | 22 March 2018 |
| | | AU 2018-200374 B2 | 19 November 2020 |
| | | AU 2018-200375 B2 | 19 November 2020 |
| | | AU 2018-200377 A1 | 22 March 2018 |
| | | AU 2018-200379 A1 | 22 March 2018 |
| | | AU 2018-200380 A1 | 22 March 2018 |
| | | AU 2018-200381 A1 | 01 February 2018 |
| | | AU 2018-207584 A1 | 09 August 2018 |
| | | AU 2018-247318 A1 | 08 November 2018 |
| | | AU 2018-260928 A1 | 06 December 2018 |
| | | AU 2019-202835 A1 | 16 May 2019 |
| | | AU 2019-203876 A1 | 20 June 2019 |
| | | AU 2021-201508 A1 | 01 April 2021 |
| | | AU 2021-202758 A1 | 27 May 2021 |
| | | AU 2023-200127 A1 | 16 February 2023 |
| | | AU 2023-214237 A1 | 31 August 2023 |
| | | CA 2859387 A1 | 20 June 2013 |
| | | CA 2868391 A1 | 10 October 2013 |
| | | CN 103974724 A | 06 August 2014 |
| | | CN 103974724 B | 30 August 2019 |
| | | CN 104114572 A | 22 October 2014 |
| | | CN 104411338 A | 11 March 2015 |
| | | CN 104870022 A | 26 August 2015 |
| | | CN 108949772 A | 07 December 2018 |
| | | CN 110201187 A | 06 September 2019 |
| | | CN 110511939 A | 29 November 2019 |
| | | CN 112390871 A | 23 February 2021 |
| | | DE 18200782 T1 | 21 October 2021 |
| | | DE 18203666 T1 | 07 October 2021 |
| | | EP 2791160 B1 | 02 March 2022 |
| | | EP 2833892 A2 | 11 February 2015 |
| | | EP 2833892 A4 | 20 July 2016 |
| | | EP 2833894 A1 | 11 February 2015 |
| | | EP 2833894 A4 | 17 August 2016 |
| | | EP 2833920 A2 | 11 February 2015 |
| | | EP 2833921 A2 | 11 February 2015 |
| | | EP 2833922 A2 | 11 February 2015 |
| | | EP 2833922 B1 | 05 December 2018 |
| | | EP 2833923 A1 | 11 February 2015 |
| | | EP 2833923 A4 | 24 February 2016 |
| | | EP 2834259 A1 | 11 February 2015 |
| | | EP 2834259 A4 | 24 August 2016 |
| | | EP 2834260 A1 | 11 February 2015 |
| | | EP 2834260 A4 | 10 August 2016 |
| | | EP 2834358 A2 | 11 February 2015 |
| | | EP 2834358 A4 | 09 March 2016 |
| | | EP 2847329 A1 | 18 March 2015 |
| | | EP 2847329 A4 | 10 August 2016 |

Form PCT/ISA/210 (patent family annex) (July 2022)

**INTERNATIONAL SEARCH REPORT**
**Information on patent family members**

International application No.
**PCT/KR2023/013302**

| Patent document cited in search report | Publication date (day/month/year) | Patent family member(s) | Publication date (day/month/year) |
|---|---|---|---|
| | | EP 2849799 A2 | 25 March 2015 |
| | | EP 2922554 A1 | 30 September 2015 |
| | | EP 2922554 B1 | 23 February 2022 |
| | | EP 2931319 A1 | 21 October 2015 |
| | | EP 2931319 A4 | 04 May 2016 |
| | | EP 2931319 B1 | 21 August 2019 |
| | | EP 2964234 A1 | 13 January 2016 |
| | | EP 3501550 A1 | 26 June 2019 |
| | | EP 3505176 A1 | 03 July 2019 |
| | | EP 4015005 A1 | 22 June 2022 |
| | | EP 4074834 A1 | 19 October 2022 |
| | | EP 4144378 A1 | 08 March 2023 |
| | | HK 1200730 A1 | 14 August 2015 |
| | | HK 1203077 A1 | 16 October 2015 |
| | | HK 1206601 A1 | 15 January 2016 |
| | | HK 1206612 A1 | 15 January 2016 |
| | | HK 1206635 A1 | 15 January 2016 |
| | | HK 1206779 A1 | 15 January 2016 |
| | | HK 1206780 A1 | 12 February 2016 |
| | | HK 1207306 A1 | 29 January 2016 |
| | | HK 1220122 A1 | 28 April 2017 |
| | | JP 2015-513912 A | 18 May 2015 |
| | | JP 2015-516143 A | 08 June 2015 |
| | | JP 2015-517995 A | 25 June 2015 |
| | | JP 2015-518704 A | 06 July 2015 |
| | | JP 2015-519040 A | 09 July 2015 |
| | | JP 2015-519881 A | 16 July 2015 |
| | | JP 2017-113029 A | 29 June 2017 |
| | | JP 2017-121239 A | 13 July 2017 |
| | | JP 2017-121240 A | 13 July 2017 |
| | | JP 2017-121244 A | 13 July 2017 |
| | | JP 2017-123847 A | 20 July 2017 |
| | | JP 2017-140048 A | 17 August 2017 |
| | | JP 2017-197545 A | 02 November 2017 |
| | | JP 2018-023373 A | 15 February 2018 |
| | | JP 2018-164458 A | 25 October 2018 |
| | | JP 2018-166528 A | 01 November 2018 |
| | | JP 2019-030327 A | 28 February 2019 |
| | | JP 2019-033757 A | 07 March 2019 |
| | | JP 2019-054813 A | 11 April 2019 |
| | | JP 2019-059793 A | 18 April 2019 |
| | | JP 2019-107022 A | 04 July 2019 |
| | | JP 2019-208511 A | 12 December 2019 |
| | | JP 2019-216733 A | 26 December 2019 |
| | | JP 2020-072670 A | 14 May 2020 |
| | | JP 2020-072766 A | 14 May 2020 |
| | | JP 2020-158508 A | 01 October 2020 |
| | | JP 2021-045163 A | 25 March 2021 |
| | | JP 2021-192606 A | 23 December 2021 |
| | | JP 2022-023036 A | 07 February 2022 |

Form PCT/ISA/210 (patent family annex) (July 2022)

**INTERNATIONAL SEARCH REPORT**
**Information on patent family members**

International application No.
**PCT/KR2023/013302**

| Patent document cited in search report | Publication date (day/month/year) | Patent family member(s) | Publication date (day/month/year) |
|---|---|---|---|
| | | JP 2022-036938 A | 08 March 2022 |
| | | JP 2022-060269 A | 14 April 2022 |
| | | JP 2022-078081 A | 24 May 2022 |
| | | JP 2022-093332 A | 23 June 2022 |
| | | JP 2022-122923 A | 23 August 2022 |
| | | JP 2023-021215 A | 10 February 2023 |
| | | JP 2023-130471 A | 20 September 2023 |
| | | JP 2023-145599 A | 11 October 2023 |
| | | JP 6189415 B2 | 30 August 2017 |
| | | JP 6348482 B2 | 27 June 2018 |
| | | JP 6424156 B2 | 14 November 2018 |
| | | JP 6426217 B2 | 21 November 2018 |
| | | JP 6449356 B2 | 09 January 2019 |
| | | JP 6553104 B2 | 31 July 2019 |
| | | JP 6921797 B2 | 18 August 2021 |
| | | JP 6946384 B2 | 06 October 2021 |
| | | JP 6953135 B2 | 27 October 2021 |
| | | US 10155029 B2 | 18 December 2018 |
| | | US 10385106 B2 | 20 August 2019 |
| | | US 10925935 B2 | 23 February 2021 |
| | | US 2013-0115272 A1 | 09 May 2013 |
| | | US 2013-0123481 A1 | 16 May 2013 |
| | | US 2013-0156849 A1 | 20 June 2013 |
| | | US 2013-0236974 A1 | 12 September 2013 |
| | | US 2013-0237594 A1 | 12 September 2013 |
| | | US 2013-0244278 A1 | 19 September 2013 |
| | | US 2013-0252281 A1 | 26 September 2013 |
| | | US 2013-0253043 A1 | 26 September 2013 |
| | | US 2014-0113959 A1 | 24 April 2014 |
| | | US 2014-0113960 A1 | 24 April 2014 |
| | | US 2014-0141067 A1 | 22 May 2014 |
| | | US 2014-0148502 A1 | 29 May 2014 |
| | | US 2014-0155472 A1 | 05 June 2014 |
| | | US 2014-0161873 A1 | 12 June 2014 |
| | | US 2014-0171485 A1 | 19 June 2014 |
| | | US 2014-0179771 A1 | 26 June 2014 |
| | | US 2021-0077634 A1 | 18 March 2021 |
| | | US 2021-0299278 A1 | 30 September 2021 |
| | | US 8999380 B2 | 07 April 2015 |
| | | US 9050297 B2 | 09 June 2015 |
| | | US 9061059 B2 | 23 June 2015 |
| | | US 9089604 B2 | 28 July 2015 |
| | | US 9095552 B2 | 04 August 2015 |
| | | US 9107886 B2 | 18 August 2015 |
| | | US 9186372 B2 | 17 November 2015 |
| | | US 9216205 B2 | 22 December 2015 |
| | | US 9220755 B2 | 29 December 2015 |
| | | US 9233141 B2 | 12 January 2016 |
| | | US 9283287 B2 | 15 March 2016 |
| | | US 9295689 B2 | 29 March 2016 |

Form PCT/ISA/210 (patent family annex) (July 2022)

**INTERNATIONAL SEARCH REPORT**
**Information on patent family members**

International application No.
**PCT/KR2023/013302**

| Patent document cited in search report | Publication date (day/month/year) | Patent family member(s) | Publication date (day/month/year) |
|---|---|---|---|
| | | US 9301993 B2 | 05 April 2016 |
| | | US 9303079 B2 | 05 April 2016 |
| | | US 9428535 B2 | 30 August 2016 |
| | | US 9504734 B2 | 29 November 2016 |
| | | US 9572896 B2 | 21 February 2017 |
| | | US 9572897 B2 | 21 February 2017 |
| | | US 9587003 B2 | 07 March 2017 |
| | | US 9597380 B2 | 21 March 2017 |
| | | US 9782462 B2 | 10 October 2017 |
| | | US 9827332 B2 | 28 November 2017 |
| | | US 9828416 B2 | 28 November 2017 |
| | | US 9878056 B2 | 30 January 2018 |
| | | WO 2013-151663 A1 | 10 October 2013 |
| | | WO 2013-151672 A2 | 10 October 2013 |
| | | WO 2013-151736 A2 | 10 October 2013 |
| WO 2018-160592 A1 | 07 September 2018 | EP 3589290 A1 | 08 January 2020 |
| | | JP 2020-510426 A | 09 April 2020 |
| | | JP 2023-052106 A | 11 April 2023 |
| | | US 11407800 B2 | 09 August 2022 |
| | | US 2018-0327471 A1 | 15 November 2018 |
| | | US 2023-0059111 A1 | 23 February 2023 |
| KR 10-2016-0091893 A | 03 August 2016 | AU 2014-340155 A1 | 05 May 2016 |
| | | AU 2014-340155 A1 | 30 April 2015 |
| | | AU 2014-340155 B2 | 01 November 2018 |
| | | AU 2019-200474 A1 | 14 February 2019 |
| | | AU 2019-200474 B2 | 26 November 2020 |
| | | AU 2021-200980 A1 | 11 March 2021 |
| | | AU 2021-200980 B2 | 20 April 2023 |
| | | AU 2023-206132 A1 | 28 September 2023 |
| | | BR 112016009077 A2 | 19 September 2017 |
| | | CA 2928078 A1 | 30 April 2015 |
| | | CL 2016000957 A1 | 09 December 2016 |
| | | CN 105813656 A | 27 July 2016 |
| | | CN 105813656 B | 15 January 2021 |
| | | CN 112618732 A | 09 April 2021 |
| | | CN 112656954 A | 16 April 2021 |
| | | EA 201690576 A1 | 31 October 2016 |
| | | EP 3060257 A1 | 31 August 2016 |
| | | EP 3060257 B1 | 24 February 2021 |
| | | EP 3871696 A1 | 01 September 2021 |
| | | JP 2016-535738 A | 17 November 2016 |
| | | JP 2019-073557 A | 16 May 2019 |
| | | JP 2020-073567 A | 14 May 2020 |
| | | JP 2021-091729 A | 17 June 2021 |
| | | JP 2022-081663 A | 31 May 2022 |
| | | JP 6646773 B2 | 14 February 2020 |
| | | JP 6851516 B2 | 31 March 2021 |
| | | JP 7046246 B2 | 01 April 2022 |
| | | KR 10-2019-0067261 A | 14 June 2019 |
| | | KR 10-2096796 B1 | 27 May 2020 |

Form PCT/ISA/210 (patent family annex) (July 2022)

**INTERNATIONAL SEARCH REPORT**
**Information on patent family members**

International application No.
**PCT/KR2023/013302**

| Patent document cited in search report | Publication date (day/month/year) | Patent family member(s) | Publication date (day/month/year) |
|---|---|---|---|
| | | MX 2016005238 A | 12 August 2016 |
| | | NZ 718817 A | 31 July 2020 |
| | | NZ 758283 A | 27 March 2020 |
| | | PE 12422016 A1 | 11 December 2016 |
| | | PE 20161242 A1 | 11 December 2016 |
| | | SG 11201602943 A | 30 May 2016 |
| | | US 10052284 B2 | 21 August 2018 |
| | | US 10493031 B2 | 03 December 2019 |
| | | US 10959953 B2 | 30 March 2021 |
| | | US 2015-0140070 A1 | 21 May 2015 |
| | | US 2017-0281542 A1 | 05 October 2017 |
| | | US 2018-0369144 A1 | 27 December 2018 |
| | | US 2020-0078299 A1 | 12 March 2020 |
| | | US 2022-0347099 A1 | 03 November 2022 |
| | | US 9629804 B2 | 25 April 2017 |
| | | WO 2015-061467 A1 | 30 April 2015 |
| KR 10-2128248 B1 | 01 July 2020 | AU 2012-267531 A1 | 19 December 2013 |
| | | AU 2012-267531 B2 | 22 June 2017 |
| | | BR 112013031553 A2 | 10 November 2020 |
| | | CA 2838069 A1 | 13 December 2012 |
| | | CA 2838069 C | 12 October 2021 |
| | | CA 3107288 A1 | 13 December 2012 |
| | | CL 2013003478 A1 | 04 July 2014 |
| | | CN 103906527 A | 02 July 2014 |
| | | CN 103906527 B | 10 July 2020 |
| | | CN 111671918 A | 18 September 2020 |
| | | CN 111671919 A | 18 September 2020 |
| | | DK 2717893 T3 | 22 July 2019 |
| | | DK 3586861 T3 | 25 April 2022 |
| | | EP 2717893 A1 | 16 April 2014 |
| | | EP 2717893 B1 | 08 May 2019 |
| | | EP 3586861 A1 | 01 January 2020 |
| | | EP 3586861 B1 | 09 February 2022 |
| | | EP 4043025 A1 | 17 August 2022 |
| | | JP 2014-523411 A | 11 September 2014 |
| | | JP 2017-014278 A | 19 January 2017 |
| | | JP 2017-203045 A | 16 November 2017 |
| | | JP 2019-048900 A | 28 March 2019 |
| | | JP 2020-186266 A | 19 November 2020 |
| | | JP 2023-087094 A | 22 June 2023 |
| | | JP 6184945 B2 | 23 August 2017 |
| | | JP 6372042 B2 | 15 August 2018 |
| | | JP 6463810 B2 | 06 February 2019 |
| | | JP 6752299 B2 | 09 September 2020 |
| | | KR 10-2020-0084048 A | 09 July 2020 |
| | | KR 10-2022-0025112 A | 03 March 2022 |
| | | ME 03491 B | 20 January 2020 |
| | | MX 2013014419 A | 23 January 2014 |
| | | MX 2019010226 A | 15 October 2019 |
| | | MX 367605 B | 28 August 2019 |

**INTERNATIONAL SEARCH REPORT**
**Information on patent family members**

International application No.
**PCT/KR2023/013302**

| Patent document cited in search report | Publication date (day/month/year) | Patent family member(s) | Publication date (day/month/year) |
|---|---|---|---|
| | | NZ 618275 A | 25 November 2016 |
| | | NZ 719345 A | 31 March 2023 |
| | | NZ 734700 A | 27 January 2023 |
| | | NZ 745686 A | 30 September 2022 |
| | | PE 07972014 A1 | 11 July 2014 |
| | | PE 20140797 A1 | 11 July 2014 |
| | | RS 59037 B1 | 30 August 2019 |
| | | RU 2013154295 A | 20 July 2015 |
| | | SI 2717893 T1 | 30 October 2019 |
| | | TR 201910686 T4 | 21 August 2019 |
| | | US 10238754 B2 | 26 March 2019 |
| | | US 10350303 B1 | 16 July 2019 |
| | | US 10413618 B2 | 17 September 2019 |
| | | US 10507249 B2 | 17 December 2019 |
| | | US 10888626 B2 | 12 January 2021 |
| | | US 11052159 B2 | 06 July 2021 |
| | | US 11185595 B2 | 30 November 2021 |
| | | US 11291734 B2 | 05 April 2022 |
| | | US 11338044 B2 | 24 May 2022 |
| | | US 11547764 B2 | 10 January 2023 |
| | | US 11730825 B2 | 22 August 2023 |
| | | US 2014-0206753 A1 | 24 July 2014 |
| | | US 2014-0294938 A1 | 02 October 2014 |
| | | US 2014-0294939 A1 | 02 October 2014 |
| | | US 2017-0239371 A1 | 24 August 2017 |
| | | US 2019-0175761 A1 | 13 June 2019 |
| | | US 2019-0192689 A1 | 27 June 2019 |
| | | US 2019-0321489 A1 | 24 October 2019 |
| | | US 2020-0085973 A1 | 19 March 2020 |
| | | US 2020-0338214 A1 | 29 October 2020 |
| | | US 2021-0060177 A1 | 04 March 2021 |
| | | US 2021-0300755 A1 | 30 September 2021 |
| | | US 2022-0040330 A1 | 10 February 2022 |
| | | US 2022-0105201 A1 | 07 April 2022 |
| | | US 2022-0111070 A1 | 14 April 2022 |
| | | US 2022-0111071 A1 | 14 April 2022 |
| | | US 2022-0111072 A1 | 14 April 2022 |
| | | US 2022-0362406 A1 | 17 November 2022 |
| | | US 2022-0378939 A1 | 01 December 2022 |
| | | US 2023-0285592 A1 | 14 September 2023 |
| | | US 2023-0285593 A1 | 14 September 2023 |
| | | US 2023-0293723 A1 | 21 September 2023 |
| | | US 9308281 B2 | 12 April 2016 |
| | | US 9597413 B2 | 21 March 2017 |
| | | WO 2012-170930 A1 | 13 December 2012 |
| | | WO 2012-170930 A9 | 03 January 2014 |
| KR 10-2022-0117133 A | 23 August 2022 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8278036 B2
- KR 102171849 B1
- KR 102014601 B1
- KR 1509456
- KR 2042993


### Non-patent literature cited in the description


- **WOLFF JA et al.** *Science,* 1990, vol. 247, 1465-8
- **SAYOUR EJ et al.** *J. Immunother. Cancer,* 2015, vol. 3, 13